# EUROPEAN PATENT APPLICATION

(11) **EP 4 382 019 A1**
(43) Date of publication of application: **12.06.2024**
(21) Application number: 22903360.0
(22) Date of filing: 05.12.2022
(51) Int. Cl.: A61B 1/00

(54) **ENDOSCOPE BUTTON MOUNTING STRUCTURE AND ENDOSCOPE**

(30) Priority: 07.12.2021 CN 202111486304
(71) Applicant: Guangzhou Red Pine Medical Instrument Co., Ltd., Guangzhou, Guangdong 510000 (CN)
(72) Inventor: YI, Feng, Guangzhou, Guangdong 510000 (CN); LI, Jing, Guangzhou, Guangdong 510000 (CN); TAN, Xiaofeng, Guangzhou, Guangdong 510000 (CN)
(74) Representative: Murgitroyd & Company
(86) International application number: PCT/CN2022/136478
(87) International publication number: WO 2023/103938

(57) **Abstract**

An endoscope button mounting structure (100) and an endoscope. The endoscope button mounting structure (100) comprises: a circuit board support (100), a circuit board (200) having a mounting surface (111) and a mounting side face (112), the mounting surface (111) being provided with a circuit board mounting part (113), and the mounting side face (112) being provided with a first snap-fit structure (114); a button support (120), the button support (120) comprising an elastic portion (121) and a mounting portion (122) which are connected to each other, the elastic portion (121) being arranged corresponding to the circuit board mounting part (113), the mounting portion (122) being provided with a second snap-fit structure (123), and the first snap-fit structure (114) being snap-fitted with the second snap-fit structure (123) to maintain a distance between the elastic portion (121) and the circuit board mounting part (113); and a button body (130), the button body (130) being arranged on the elastic portion (121), and the button body (130) being configured to drive the elastic portion (121) to generate elastic deformation relative to the mounting portion (122) in a direction close to the circuit board mounting part (113) under the action of an external force. According to the endoscope button mounting structure (100) and the endoscope, the structure is simple, the convenience of mounting a button on a handle can be effectively improved, and the convenience of operation for a medical worker can also be improved.

## Description

### TECHNICAL FIELD

The present application relates to the technical field of medical devices, and in particular, to an endoscope button mounting structure and an endoscope.

### BACKGROUND

With the development of a medical technology, an endoscope technology has emerged. An endoscope is configured to visually inspect hard-to-reach sites such as organs in a human body to observe lesions, which is a detection instrument that integrates traditional optics, ergonomics, precision machinery, modern electronics, mathematics, software and the like, and includes an image sensor, an optical lens, a lighting source, a mechanical apparatus, and the like. The endoscope may enter a lumen of the human body through a natural opening to examine a lumen in communication with the outside (such as a digestive tract, a respiratory tract, and a urinary tract), or may be inserted into closed lumen (such as a chest, an abdominal cavity, and a joint cavity) through an incision for examination. Medical endoscopes have been widely used in examinations in various departments, which may be divided into endoscopes for ear, nose and throat (ENT), gastrointestinal endoscopes, ureteroscopes, arthroscopes, and the like according to sites to which the medical endoscopes may reach in the human body.

In the conventional art, a medical endoscope mainly includes an insertion portion and a handle. The insertion portion enters the human body's organs, while the handle is operated outside the human body by a medical worker. In order to meet examination requirements, the endoscope has more and more functions, and there are more and more function buttons corresponding thereto. However, operations of mounting these buttons are complicated, leading to low reliability and cumbersome operations.

### SUMMARY

Accordingly, it is necessary to provide an endoscope button mounting structure and an endoscope, which have a simple structure, and can effectively improve convenience of mounting a button on a handle and improve the convenience of operation of a medical worker.

An endoscope button mounting structure includes: a circuit board support, a circuit board having a mounting surface and a mounting side surface, the mounting surface being provided with a circuit board mounting part, and the mounting side surface being provided with a first engaging structure; a button support including an elastic portion and a mounting portion that are connected to each other, the elastic portion being arranged corresponding to the circuit board mounting part, the mounting portion being provided with a second engaging structure, and the first engaging structure being engaged with the second engaging structure to maintain a distance between the elastic portion and the circuit board mounting part; and a button body arranged on the elastic portion, and the button body being configured to drive the elastic portion to generate elastic deformation relative to the mounting portion in a direction approaching the circuit board mounting part under the action of an external force.

According to the above endoscope button mounting structure, during the mounting, firstly, the circuit board is mounted at the circuit board mounting part of the circuit board support. Then, the button body is arranged on the elastic portion, and the button body can drive the elastic portion to generate deformation towards the circuit board mounting part. Since the first engaging structure is engaged with the second engaging structure, it is beneficial to improve the convenience of mounting the button body, and maintain the distance between the elastic portion and the circuit board mounting part. A medical worker may directly press the button body to cause the elastic portion to deform, performing a touch operation on the circuit board. This direct pressing manner is realized by a simple structure, and the reliability is strong, which is beneficial to improve the convenience of operation of the medical worker.

In an embodiment, the first engaging structure is an engaging groove, and the second engaging structure is an engaging protrusion fitted into the engaging groove.

In an embodiment, the first engaging structure includes a first boss and a second boss. The first boss and the second boss are spaced apart to form the engaging groove.

In an embodiment, the first boss is located on a side of the second boss away from the elastic portion. A side of the second boss adjacent to the first boss is provided with a introducing inclined surface configured to guide the engaging protrusion into the engaging groove.

In an embodiment, the first boss is located on a side of the second boss away from the elastic portion. A side of the second boss away from the first boss protrudes relative to the mounting surface. The elastic portion is provided with an avoiding hole. The second boss extends through the avoiding hole.

In an embodiment, an abutting block is provided on an inner wall of the avoiding hole. The abutting block abuts against the second boss.

In an embodiment, the second boss is located on a side portion of the circuit board mounting part, and is configured to abut against a circuit board. The mounting surface is provided with a limiting protrusion. The limiting protrusion is located on an end portion of the circuit board mounting part, and configured to abut against the circuit board.

In an embodiment, a contact is provided on a surface of the elastic portion facing the circuit board mounting part.

In an embodiment, a contact is provided on a surface of the button body facing the circuit board mounting part.

In an embodiment, the mounting portion and the elastic portion are integrally formed.

In an embodiment, the elastic portion and the button body are integrally formed.

In an embodiment, the button body is provided with a lightening cavity.

In an embodiment, the mounting side surface is provided with a mounting hole. A connecting member extends through the mounting hole to cause the circuit board support to be connected to an endoscope housing.

In an embodiment, a plurality of first engaging structures are provided. The plurality of first engaging structures are spaced apart in an extending direction of the circuit board mounting part. A plurality of button supports are provided. The first engaging structures and the second engaging structures of the button supports are in snap fit with each other in one-to-one correspondence. A plurality of button bodies are provided. The button bodies are arranged on the elastic portions of the button supports in one-to-one correspondence.

An endoscope includes a circuit board and the endoscope button mounting structure according to any one of the above embodiments. The circuit board is arranged at the circuit board mounting part.

According to the above endoscope, during the mounting, firstly, the circuit board is mounted at the circuit board mounting part of the circuit board support. Then, the button body is arranged on the elastic portion, and the button body can drive the elastic portion to generate deformation towards the circuit board mounting part. Since the first engaging structure is engaged with the second engaging structure, it is beneficial to improve the convenience of mounting the button body and maintain the distance between the elastic portion and the circuit board mounting part. A medical worker may directly press the button body to cause the elastic portion to deform, performing a touch operation on the circuit board. This direct pressing manner is realized by a simple structure, and the reliability is strong, which is beneficial to improve the convenience of operation for the medical worker.

### BRIEF DESCRIPTION OF THE DRAWINGS

The accompanying drawings forming part of the present application are intended to provide further understanding of the present application. Exemplary embodiments of the present application and descriptions thereof are intended to explain the present application, and do not constitute any inappropriate limitation on the present application.

In order to more clearly illustrate the technical solutions in embodiments of the present application, the accompanying drawings used in the description of the embodiments will be briefly introduced below. It is apparent that, the accompanying drawings in the following description are only some embodiments of the present application, and other drawings can also be obtained by those of ordinary skill in the art from the provided drawings without creative efforts.
FIG. 1 is a first schematic perspective view of an endoscope mounting structure according to an embodiment.
FIG. 2 is a second schematic perspective view of the endoscope mounting structure according to an embodiment.
FIG. 3 is a schematic perspective view of a circuit board support according to an embodiment.

### Illustration for reference signs:

100: endoscope button mounting structure; 110: circuit board support; 111: mounting surface; 1111: limiting protrusion; 112: mounting side surface; 1121: mounting hole; 113: circuit board mounting part; 114: first engaging structure; 1141: first boss; 1142: second boss; 1143: introducing inclined surface; 120: button support; 121: elastic portion; 1211: avoiding hole; 1212: abutting block; 122: mounting portion; 123: second engaging structure; 130: button body; 131: contact; 200: circuit board.

### DETAILED DESCRIPTION OF THE EMBODIMENTS

In order to make the above objects, features, and advantages of the present application more obvious and understandable, specific implementations of the present application are described in detail below with reference to the accompanying drawings. In the following description, many specific details are set forth in order to fully understand the present application. However, the present application can be implemented in many other ways different from those described herein, and those skilled in the art can make similar improvements without departing from the connotation of the present application. Therefore, the present application is not limited by the specific embodiments disclosed below.

In the description of the present application, it should be understood that the orientation or position relationships indicated by the terms "central", "longitudinal", "transverse", "length", "width", "thickness", "upper", "lower", "front", "rear", "left", " right", "vertical", "horizontal", "top", "bottom", "inner", "outer", "clockwise", "counterclockwise", "axial", "radial", "circumferential", and the like are based on the orientation or position relationships shown in the accompanying drawings and are intended to facilitate the description of the present application and simplify the description only, rather than indicating or implying that the device or element referred to must have a particular orientation or be constructed and operated in a particular orientation, and therefore are not to be interpreted as limiting the present application.

In addition, the terms "first" and "second" are used for descriptive purposes only, which cannot be construed as indicating or implying a relative importance, or implicitly specifying the number of the indicated technical features. Therefore, the features defined by "first" and "second" may explicitly or implicitly include at least one of the features. In the description of the present application, "a plurality of" means two or more, such as two or three, unless specifically stated otherwise.

In the present application, unless otherwise specifically stated and defined, the terms such as "mounting", "coupling", "connection", and "fixation" should be understood in a broad sense, such as, a fixed connection, a detachable connection, or an integral connection; a mechanical connection, or an electrical connection; a direct connection, an indirect connection through an intermediate medium; an internal connection between two elements, or interaction between two elements, unless otherwise specifically defined. For those of ordinary skill in the art, the specific meanings of the foregoing terms in the present application can be understood on a case-by-case basis.

In the present application, unless otherwise explicitly specified and defined, the expression a first feature being "on" or "under" a second feature may be the case that the first feature is in direct contact with the second feature, or the first feature is in indirect contact with the second feature via an intermediate medium. Furthermore, the expression a first feature being ""over", "above", or "on top of' a second feature may be the case that the first feature is directly above or obliquely above the second feature, or only means that the level of the first feature is higher than that of the second feature. The expression a first feature being "below", "underneath", or "under" a second feature may be the case that the first feature is directly underneath or obliquely underneath the second feature, or only means that the level of the first feature is lower than that of the second feature.

It should be noted that when one element is referred to as "fixed to" or "arranged on" another element, it may be directly disposed on the other element or an intermediate element may exist. When one element is considered to be "connected to" another element, it may be directly connected to the other element or an intermediate element may co-exist. The terms "perpendicular", "horizontal", "left", "right", and similar expressions used herein are for illustrative purposes only, and do not represent the unique implementation.

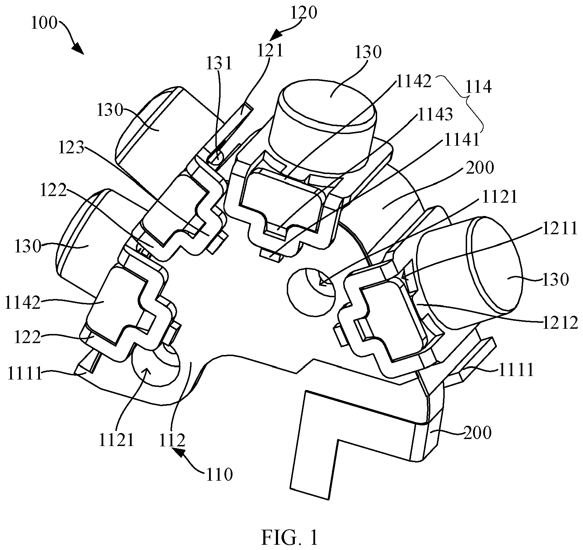

In an embodiment, referring to FIGS. 1, 2, and 3, an endoscope button mounting structure 100 includes a circuit board support 110, a button support 120, and a button body 130. A circuit board 200 has a mounting surface 111 and a mounting side surface 112. The mounting surface 111 is provided with a circuit board mounting part 113. The mounting side surface 112 is provided with a first engaging structure 114. The button support 120 includes an elastic portion 121 and a mounting portion 122 that are connected to each other. The elastic portion 121 is arranged corresponding to the circuit board mounting part 113. The mounting portion 122 is provided with a second engaging structure 123. The first engaging structure 114 is engaged with the second engaging structure 123 to maintain a distance between the elastic portion 121 and the circuit board mounting part 113. The button body 130 is arranged on the elastic portion 121. The button body 130 is configured to drive the elastic portion 121 to generate elastic deformation relative to the mounting portion 122 in a direction approaching the circuit board mounting part 113 under the action of an external force.

According to the above endoscope button mounting structure 100, during the mounting, firstly, a circuit board 200 is mounted at the circuit board mounting part 113 of the circuit board support 110. Then, the button body 130 is arranged on the elastic portion 121 and the button body 130 can drive the elastic portion 121 to generate deformation towards the circuit board mounting part 113. Since the first engaging structure 114 is engaged with the second engaging structure 123, it is beneficial to improve the convenience of mounting the button body 130, and maintain the distance between the elastic portion 121 and the circuit board mounting part 113 of the circuit board 200. A medical worker may directly press the button body 130 to cause the elastic portion 121 to deform, performing a touch operation on the circuit board 200. This direct pressing manner is realized by a simple structure, and the reliability is strong, which is beneficial to improve the convenience of operation of the medical worker.

It should be noted that the button body 130 is configured to drive the elastic portion 121 to generate elastic deformation relative to the mounting portion 122 in the direction approaching the circuit board mounting part 113 under the action of the external force, thereby performing a touch or press operation on the circuit board 200 and realizing signal conversion.

Optionally, the first engaging structure 114 may be engaged with the second engaging structure 123 in such a way that: the first engaging structure 114 is an engaging groove and the second engaging structure 123 is an engaging protrusion, or the first engaging structure 114 is an engaging protrusion and the second engaging structure 123 is an engaging groove, or in other engaging manners.

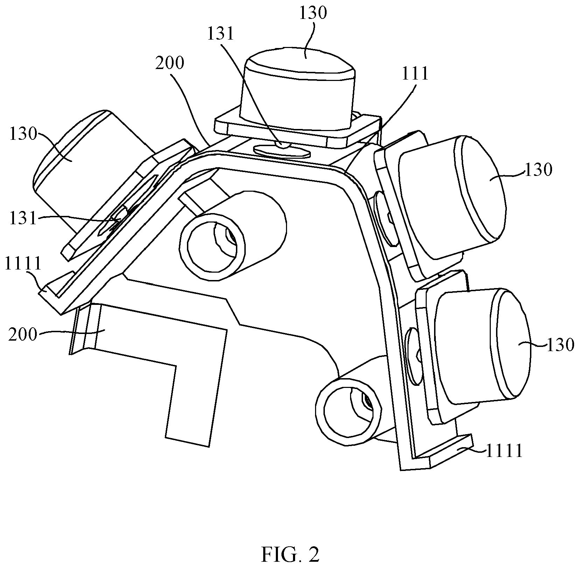

Specifically, referring to FIGS. 1 and 2, the first engaging structure 114 is an engaging groove, and the second engaging structure 123 is an engaging protrusion fitted into the engaging groove. In this way, during the mounting, the engaging protrusion is pressed to be engaged into the engaging groove for engaging fit, which realizes a connection between the circuit board support 110 and the button support 120. The structure is simple, and the connection is reliable, thereby improving reliability of use of the endoscope button mounting structure 100.

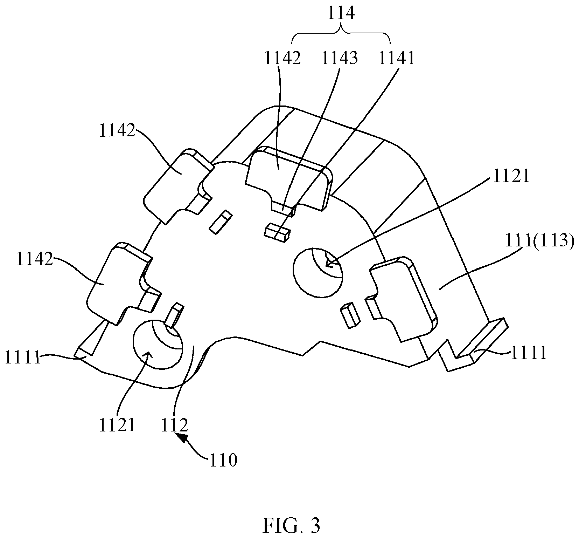

In an embodiment, referring to FIGS. 1 and 3, the first engaging structure 114 includes a first boss 1141 and a second boss 1142. The first boss 1141 and the second boss 1142 are spaced apart to form the engaging groove. In this way, the first boss 1141 and the second boss 1142 play a role in contacting and locking the second engaging structure 123, which is beneficial to improve the stability of the connection between the first engaging structure 114 and the second engaging structure 123, thereby improving the stability of the mounting of the button body 130, and improving overall use quality of the endoscope button mounting structure 100.

Further, referring to FIG. 1, the first boss 1141 is located on a side of the second boss 1142 away from the elastic portion 121. A side of the second boss 1142 adjacent to the first boss 1141 is provided with an introducing inclined surface 1143. The introducing inclined surface 1143 is configured to guide the engaging protrusion into the engaging groove. In this way, the introducing inclined surface 1143 can guide the second engaging structure 123 into the engaging groove, which is beneficial to improve the convenience of mounting the button body 130, thereby improving overall mounting efficiency of the endoscope button mounting structure 100.

In an embodiment, referring to FIG. 1, the first boss 1141 is located on the side of the second boss 1142 away from the elastic portion 121. A side of the second boss 1142 away from the first boss 1141 protrudes relative to the mounting surface 111. The elastic portion 121 is provided with an avoiding hole 1211. The second boss 1142 extends through the avoiding hole 1211. In this way, the second boss 1142 extends through the avoiding hole 1211, which facilitates the engaging protrusion to be smoothly engaged into the engaging groove for engaging fit, thereby improving stability of the connection between the button support 120 and the circuit board support 110, and improving use reliability and overall quality of the endoscope button mounting structure 100.

Further, referring to FIG. 1, an abutting block 1212 is provided on an inner wall of the avoiding hole 1211. The abutting block 1212 abuts against the second boss 1142. In this way, the abutting block 1212 abuts against the second boss 1142, which can fix and limit the mounting portion 122 and maintain stability of the mounting portion 122, thereby improving stability of the connection between the first engaging structure 114 and the second engaging structure 123, ensuring stability of the mounting of the button body 130, and improving overall quality and use reliability of the endoscope button mounting structure 100.

In an embodiment, referring to FIGS. 1 and 3, the second boss 1142 is located on a side portion of the circuit board mounting part 113. The second boss 1142 is configured to abut against the circuit board 200. The mounting surface 111 is provided with a limiting protrusion 1111. The limiting protrusion 1111 is located on an end portion of the circuit board mounting part 113. The limiting protrusion 1111 is configured to abut against the circuit board 200. In this way, the second boss 1142 can limit the circuit board 200 to restrict the circuit board 200 from moving to the side portion of the circuit board mounting part 113, and fix the circuit board 200 to the circuit board mounting part 113 on the mounting surface 111. On the other hand, the limiting protrusion 1111 can limit the circuit board 200 to prevent the circuit board 200 from moving to the end portion of the circuit board mounting part 113, and further fix the circuit board 200 to the circuit board mounting part 113 on the mounting surface 111, thereby improving stability of the mounting of the circuit board 200.

In an embodiment, referring to FIG. 1, a contact 131 is provided on a surface of the elastic portion 121 facing the circuit board mounting part 113. In this way, by pressing the button body 130. By pressing the button body 130, the contact 131 may perform a touch or press operation on the circuit board 200, thereby reducing a pressing distance, improving the convenience of use of the button body 130, and ensuring use efficiency of the endoscope.

In another embodiment, a contact 131 is provided on a surface of the button body 130 facing the circuit board mounting part 113. In this way, similarly, by pressing the button body 130, the contact 131 may perform a touch or press operation on the circuit board 200, thereby reducing a pressing distance, improving the convenience of use of the button body 130, and ensuring using efficiency of the endoscope.

Optionally, the mounting portion 122 and the elastic portion 121 may be connected to each other by bonding, welding, snap fit, inserting, or the like. Alternatively, the mounting portion 122 and the elastic portion 121 are integrally formed.

Specifically, referring to FIGS. 1 and 2, the mounting portion 122 and the elastic portion 121 are integrally formed. In this way, the structure is simple, and the manufacturing is easy, which is beneficial to ensure connection stability and use reliability of the mounting portion 122 and the elastic portion 121, reducing manufacturing costs, and improving economic benefits. Although this embodiment only provides a specific manner of connecting the mounting portion 122 and the elastic portion 121, but which is not limited thereto.

Optionally, the button body 130 and the elastic portion 121 may be connected to each other by bonding, welding, snap fit, inserting, or the like. Alternatively, the button body 130 and the elastic portion 121 are integrally formed.

Specifically, referring to FIGS. 1 and 2, the elastic portion 121 and the button body 130 are integrally formed. In this way, the structure is simple, and the manufacturing is easy, which is beneficial to ensure connection stability and use reliability of the button body 130 and the elastic portion 121, reducing manufacturing costs, and improving economic benefits. Although this embodiment only provides a specific manner of connecting the button body 130 and the elastic portion 121, but which is not limited thereto.

Preferably, the button body 130, the mounting portion 122, and the elastic portion 121 are integrally formed by injection molding, which is beneficial to reduce manufacturing costs and improve overall structural stability.

In an embodiment, referring to FIGS. 1 and 2, the button body 130 is provided with a lightening cavity. This is beneficial to reduce weight of the button body 130, facilitates rebound of the elastic portion 121, prolongs the service life of the elastic portion 121, and improves use reliability of the elastic portion 121, thereby improving the overall quality of the endoscope button mounting structure 100.

In an embodiment, referring to FIGS. 1 and 2, the mounting side surface 112 is provided with a mounting hole 1121. A connecting member extends through the mounting hole 1121 to cause the circuit board support 110 to be connected to an endoscope housing. This is beneficial to improve the convenience of mounting the circuit board support 110 and the endoscope housing, thereby improving the overall quality of the endoscope button mounting structure 100.

In an embodiment, referring to FIGS. 1 and 2, a plurality of first engaging structures 114 are provided. The plurality of first engaging structures 114 are spaced apart in an extending direction of the circuit board mounting part 113. A plurality of button supports 120 are provided. The first engaging structures 114 and the second engaging structures 123 of the button supports 120 are in snap fit with each other in one-to-one correspondence. A plurality of button bodies 130 are provided. The button bodies 130 are arranged on the elastic portions 121 of the button supports 120 in one-to-one correspondence. In this way, the plurality of button bodies 130 can be mounted on the circuit board support 110, and then a plurality of different operating functions can be provided, which is beneficial to improve the convenience of use of the endoscope.

In an embodiment, an endoscope (not shown) includes a circuit board 200 and the endoscope button mounting structure 100 according to the above embodiments. The circuit board 200 is arranged at the circuit board mounting part 113.

According to the above endoscope, during the mounting, firstly, the circuit board 200 is mounted at the circuit board mounting part 113 of the circuit board support 110. Then, the button body 130 is arranged on the elastic portion 121, and the button body 130 can drive the elastic portion 121 to generate deformation towards the circuit board mounting part 113. Since the first engaging structure 114 is engaged with the second engaging structure 123, it is beneficial to improve the convenience of mounting the button body 130, and maintain the distance between the elastic portion 121 and the circuit board mounting part 113 of the circuit board 200. The medical worker may directly press the button body 130 to cause the elastic portion 121 to deform, performing a touch operation on the circuit board 200. This direct pressing manner is realized by a simple structure, and the reliability is strong, which is beneficial to improve the convenience of operation of the medical worker.

The technical features in the above embodiments may be randomly combined. For concise description, not all possible combinations of the technical features in the above embodiments are described. However, all the combinations of the technical features are to be considered as falling within the scope described in this specification provided that they do not conflict with each other.

The above embodiments only describe several implementations of the present application, and their description is specific and detailed, but cannot therefore be understood as a limitation on the patent scope of the invention. It should be noted that those of ordinary skill in the art may further make variants and improvements without departing from the conception of the present application, and these all fall within the protection scope of the present application. Therefore, the patent protection scope of the present application should be subject to the appended claims.

## Claims

1. An endoscope button mounting structure, comprising:
a circuit board support, the circuit board having a mounting surface and a mounting side surface, the mounting surface being provided with a circuit board mounting part, and the mounting side surface being provided with a first engaging structure;
a button support comprising an elastic portion and a mounting portion that are connected to each other, the elastic portion being arranged corresponding to the circuit board mounting part, the mounting portion being provided with a second engaging structure, and the first engaging structure being engaged with the second engaging structure to maintain a distance between the elastic portion and the circuit board mounting part; and a button body arranged on the elastic portion, and the button body being configured to drive the elastic portion to generate elastic deformation relative to the mounting portion in a direction approaching the circuit board mounting part under the action of an external force.

2. The endoscope button mounting structure according to claim 1, wherein the first engaging structure is an engaging groove, and the second engaging structure is an engaging protrusion fitted into the engaging groove.

3. The endoscope button mounting structure according to claim 2, wherein the first engaging structure comprises a first boss and a second boss; the first boss and the second boss being spaced apart to form the engaging groove.

4. The endoscope button mounting structure according to claim 3, wherein the first boss is located on a side of the second boss away from the elastic portion; and a side of the second boss adjacent to the first boss is provided with an introducing inclined surface configured to guide the engaging protrusion into the engaging groove.

5. The endoscope button mounting structure according to claim 3, wherein the first boss is located on a side of the second boss away from the elastic portion; a side of the second boss away from the first boss protrudes relative to the mounting surface; the elastic portion is provided with an avoiding hole; and the second boss extends through the avoiding hole.

6. The endoscope button mounting structure according to claim 5, wherein an abutting block is provided on an inner wall of the avoiding hole; and the abutting block abuts against the second boss.

7. The endoscope button mounting structure according to claim 5, wherein the second boss is located on a side portion of the circuit board mounting part, and is configured to abut against a circuit board; and the mounting surface is provided with a limiting protrusion; the limiting protrusion being located on an end portion of the circuit board mounting part, and configured to abut against the circuit board.

8. The endoscope button mounting structure according to claim 1, wherein a contact is provided on a surface of the elastic portion facing the circuit board mounting part; or
a contact is provided on a surface of the button body facing the circuit board mounting part.

9. The endoscope button mounting structure according to claim 1, wherein the mounting portion and the elastic portion are integrally formed.

10. The endoscope button mounting structure according to claim 1, wherein the elastic portion and the button body are integrally formed.

11. The endoscope button mounting structure according to claim 1, wherein the button body is provided with a lightening cavity.

12. The endoscope button mounting structure according to claim 1, wherein the mounting side surface is provided with a mounting hole; a connecting member extends through the mounting hole to cause the circuit board support to be connected to an endoscope housing.

13. The endoscope button mounting structure according to any one of claims 1 to 12, wherein a plurality of first engaging structures are provided; the plurality of first engaging structures being spaced apart in an extending direction of the circuit board mounting part; a plurality of button supports are provided; the first engaging structures and the second engaging structures of the button supports are in snap fit with each other in one-to-one correspondence; and a plurality of button bodies are provided; the button bodies are arranged on the elastic portions of the button supports in one-to-one correspondence.

14. An endoscope, comprising:
a circuit board; and
the endoscope button mounting structure according to any one of claims 1 to 13, wherein the circuit board are arranged at the circuit board mounting part.
